# EUROPEAN PATENT SPECIFICATION

(11) **EP 2 215 970 B1**
(45) Date of publication and mention of the grant of the patent: **29.02.2012**
(21) Application number: 09179059.2
(22) Date of filing: 14.12.2009
(51) Int. Cl.: A61B 10/00, B01L 3/00, B01L 9/00

(54) **Preserving container**
Konservierungsbehälter
Récipient de protection

(30) Priority: 05.02.2009 DE 202009001433 U
(43) Date of publication of application: 11.08.2010
(73) Proprietor: EPPENDORF AG, 22339 Hamburg (DE)
(72) Inventor: Schmiedl, Dieter, 04626, Schmölln (DE)
(74) Representative: Becker Kurig Straus

(56) References cited:
- EP-A1- 0 466 009
- WO-A1-2004/022235
- WO-A2-2008/095458
- US-B1- 6 569 676

## Description

### Field of application

The invention relates to a preserving container for securing a large amount of samples of microbiologic, virologic, genetic, medical, veterinary medical, forensic, criminal-listic and technical origin, which have been collected with a system essentially on the basis of a modified micro litre pipette tip.

Said preserving container is a component of an apparatus system for collecting/ sampling, identifying, storing, and further processing and providing samples, respectively.

### State of the art

Collecting systems having swabs/cotton wool swabs as actual sample carrier are common in forensics and criminalistics. These swabs mostly consist of a shaft/stick made of wood, metal or plastics to which a piece of cotton wool is provided on one or both ends.

Said sample carriers are typically stored in specific cardboard boxes having an integrated cotton wool shaft attachment, e.g. according to US 6,171,260 and US 6,085,907. However, when using such cardboard boxes a large portion of the available storage room remains unused. Also, the handling of the cardboard boxes with respect to folding, closing, inscribing and transporting does not meet the requirements of today's working economy, neither in the process of securing evidence nor in the laboratory.

Separately drying the individual mostly long-shafted swabs confers a risk for contamination and is work- and time consuming.

In EP 1 596 720 a closable container having a cap is described, which container can receive a wet swab in an attachment and can dry said swab in a tube system by means of ventilation holes.

In the sealed/closed container according to DE 20 2007 001 898 inserted wet biological samples can be dried by guaranteeing an air exchange via pores in the container wall or in the closure.

EP 1 234 165 also presents a sample container for preserving and dry storing of DNA/RNA containing samples, which is essentially a container having a specific design and a sample located inside. For drying the sample hygroscopic substances are required, which are fixed in the sample container in various ways and which remain therein permanently.

DE 696 30 819 describes a closed container comprising a drying agent added to a container material.

A flexible marsupial storage container having - to a specific degree - water and vapour permeable walls is known from DE 601 14 513. WO 2004/022235 discloses a preserving container comprising a sample container, closable with a diffusion permeable lid, and a sealing cover.

Finally, German utility model 20 2008 013 218 describes a sample carrier on the basis of a micro litre pipette tip. This sample carrier can be handled by means of a forensic pipette according to German utility model 20 2008 013 219, which also allows wetting the cotton wool head.

A sample is collected from a substrate independently of the used swab system either by dry or wet rubbing off the surface/substrate and wiping and is then transferred to further studying and analysis.

Due to the fact that usually the further analysis in the lab is not carried out promptly after sample collection a temporary storage is required.

Also, after analysis the remainders of the original samples must be stored for subsequent or additional analysis, at least until the case is closed. The storing period varies; it often lasts for several years.

According to German utility model 20 2008 017 181 aliquots may be taken from dried and already stored sample carriers. Depending on the employed method these original swabs must often be slightly wetted and after aliquotting they must be accurately dried immediately.

In the context of this invention the secure storage and preservation of samples obtained by aliquotting is of particular interest.

In general, the purpose of securing samples to be used as evidence resides in retrieving DNA material in appropriate purity and molecular integrity. Unfortunately, DNA samples colleted by means of swabs or micro litre pipette tips are merely durable for a short time, since they will be easily degraded by omnipresent endonucleases. If exposed to said nucleases, most of which are of bacterial origin, DNA will be degraded to short fragments. Nearly every collected DNA sample is contaminated by a plurality of different microbes. In the extreme the DNA can be irrecoverably destroyed resulting in the complete loss of the sample. The DNA on the sample carrier which is often present in only low quantities is additionally compromised by various environmental effects. In order to avoid degradation of the DNA the sample must be most accurately handled. The sample material must unconditionally be protected from the most important risk factors, namely contamination, humidity, heat and UV-light.

All DNA containing samples collected for the purpose of an analytical study/investigation are of high juridical relevance. Such samples must therefore be protected from qualitative and quantitative losses or falsifications of all sorts. For economic reasons also the storing of the sample shall be suitable for routine work in this respect.

Besides the already mentioned risks, which result from storage conditions and contamination dangers, the possibility of a sample mix up or manipulation may not be overlooked. Sample identification per se, be it inscription, numbering, barcode or the like, is often not directly arranged on the sample material and is mostly mounted outside of the sealing of the sample container in a non forgery-proof way.

At present large volume capacities remain unused in all known methods for dry storing. Also, the total weight as a result of incorporated hygroscopic substances and of the water remaining therein is disproportionately high. This means that storage and material capacities are wasted.

Considering the mentioned risks and acknowledging the relevant state of the art it would be desirable to provide a sample storage and preservation system by which large sample amounts can be handled in a routine and safe way.

### Problem

The problem underlying the present invention is therefore to be seen in the development of a sample container for receiving, transporting, drying and long term storing of a plurality of sample carriers, particularly for forensic and criminalistic purposes. In this respect the use of sample carriers on the basis of the newly designed micro litre pipette tips with applied cotton wool heads according to German utility model 20 2008 013 218 is particularly envisioned. Yet, also the widely used shaft swabs shall be used.

### Solution to the problem

The above problem has been solved by providing a preserving container, wherein a sample container closable with a lid is arranged in a storage container provided with a sealing cover, wherein the sample container has separated cavities, in which sample carriers can be positioned without contacting any wall- and bottom of the sample container, and wherein the sample container and the lid are made of diffusion permeable material, and wherein the storage container as well as its cover are made of diffusion impermeable material.

In a further embodiment of the invention swabtips, alternatively also shaft swabs as sample carrier, can be arranged in the cavities. Also, the sample container is dimensionally adjusted for taking up swabtips and/or shaft swabs, respectively. Also, specific arrangements are provided for a circulation of air between the individual cavities, namely corresponding clearances.

In still another embodiment of the invention the sample container has at least one cavity, optimally 96 or more than 96 cavities.

In another embodiment of the invention all cavities can be sealed using a central sealable lid.

In still another embodiment of the invention the storage container comprises a plurality of sample containers.

In yet a further embodiment of the present invention a humidity indicator is located within the container or on an outside surface of the container.

To implement the invention it is proposed to use sample carriers on the basis of a micro litre pipette tip according to German utility model 20 2008 013 218 for the purpose mentioned above. The sample carriers (swabs/swabtips) described therein can be plugged on simple attachments or laboratory accepted micro litre pipettes. The forensic pipette described in German utility model 20 2008 013 219 can be used advantageously for wetting and comfortable handling of such sample carriers. A further advantage is a permanent identification (transponder, barcode or the like) according to DE 20 2008 014 965 which may be directly mounted on the sample carrier. A highest possible safety from mixing up would thus be guaranteed.

The subject matter of the present invention is a preserving container in form of a collecting box for receiving a defined number of sample carriers, namely swabs/swabtips and alternatively shaft swabs, respectively. Accommodation of swabtips is preferred based on their minimal dimensions. Accommodation of shaft swabs is of particular concern due to their wide spreading.

According to the invention a sample container is diverted into separated individual cavities. At least one cavity must be present. Swabtips and shaft swabs, respectively, can each be positioned in the dimensionally adjusted individual cavities without the cotton wool head contacting any wall and bottom of said container.

The sample container is non-detachably connected to a lid, which may close and seal all cavities simultaneously. Thus, any type of contamination can be sufficiently avoided.

The closed and sealed sample container is accommodated in a covered storage container. If necessary, the latter can accommodate more than one sample container. While the sample container, including its lid, is made of light and stable cardboard or of another material having equivalent attributes, the storage container and its cover are made diffusion impermeable. To this end plastics are preferred materials.

Arranging or providing hygroscopic drying agents is not envisioned. Although drying agents bind water in the sample chamber by absorptive processes, they do not remove it from the closed system. Since adsorptive and desorptive processes are always in equilibrium, changing humidity levels must be expected in air tight containers within a longer storage duration, brought about by aging processes of the drying agent and possible temperature fluctuations. In this respect sweat or condensation water on cold sides and also the possibility of local development of mould fungus must be expected. Moreover, the weight of adsorption agent would recognisably and thus adversely add up.

The term sample carrier as used herein comprises common swabs/cotton wool swabs as actual sample carrier. They consist of a shaft/stick mostly made of wood, metal or plastics to which a piece of cotton wool is provided on one or both ends. The term cardboard in this context comprises all closable containers, like cardboard boxes, containers, cartons, tins, casings/sleeves or the like, which are mainly made of cellulose or other derivatives.

All products made of cellulose belong thereto (for example paper and cardboard), directly pressed plant fibres (for example cotton wool, jute, flax, hemp, sisal or ramie), hemicelluloses (for example crop straw), cellulose based chemical fibres (for example viscose fibres, cellulose fibres, acetate- and triacetate-fibres) or mixtures thereof.

Closed cardboard containers made of these materials may easily be penetrated by gaseous molecules, hence also by water molecules. Water molecules diffuse cellulose walls until equilibrium with the environmental compartment air is set. However, this rule also applies in the reverse direction, in particular when the compartment air is more humid than the enclosed air in the sample containers.

The preserving container according to the invention (storage container having one or more sample containers laying inside) is besides its actual purpose also usable as reservoir- and working container for common 96 pieces of swabtips, wherein each rack can be used successively.

The working dimension of 8 by 12 tests, which has been successfully used for years in analytics (for example in enzyme immunoassays), is recommended for routine work with swabtips. 96 DNA sample carriers per rack can be simultaneously processed, transported, dried, documented and stored.

Also preserving containers having merely one chamber, having a round, oval or angled form, and/or having tubes or sleeves in the widest sense for positioning single samples are envisioned.

The described features of the device allow the following principal working scheme:
- By using a forensic pipette e.g. swabtips are taken from a storage container for collecting samples to be used as evidence.
- Swabtips containing the collected DNA-samples, mostly more or less wet, are successively positioned in a cardboard container of the same type, serving as working box. After inserting the last serial sample or after sample number 96 the cardboard lid is closed and sealed.

The samples immediately start drying in the cardboard container, wherein the drying level eventually depends on the humidity of the environmental air surrounding said container.
- In the lab the above samples, which further remain in the closed and sealed working boxes, are finally dried according to proven methods by professionally treating DNA caringly.
- By positioning the dried sample containers in closable, water proof and accurately fitting plastic boxes, with or without humidity indicator, said dried samples can be stored at room temperature in a time-independent way. Low space requirements allow a reasonable exploitation of spatial resources.

The preserving container according to the present invention can be used repeatedly. Thus, said container can be used in an economically reasonable way. The proposed materials are ecologically compatible since they are biologically degradable.

In case the racks are equipped with 8 x 12 samples, serial working with 96 pieces will be possible. However, this arrangement is not compulsive. Yet, said arrangement allows use of established micro litre pipettes up to use of multi-channel micro litre pipettes.

The loaded preserving containers require only few space and are very light, since they contain neither drying agents nor water.

Even in case of temperature fluctuations no condensation water forms in the interior of the individual cavities. Abandonment of additional adsorption agents is economically advantageous. Contaminations of the stored samples with drying agent powders are excluded. The desired light protection results from the selected material, which may be cardboard or paper or any other material with comparable attributes and preferably cellulose.

### Example

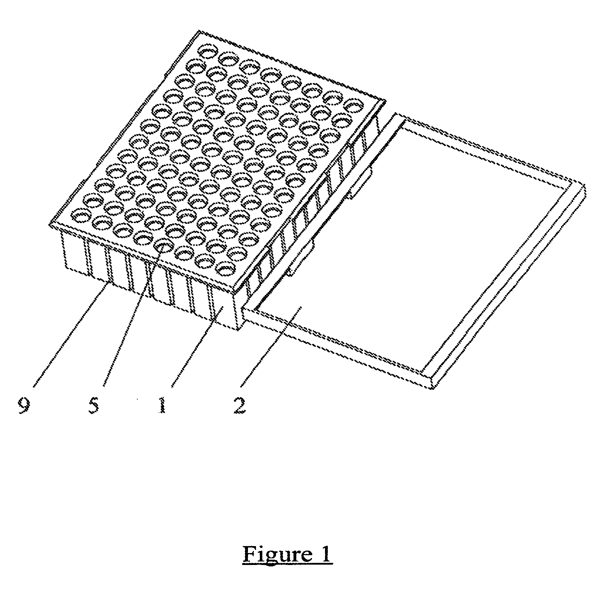

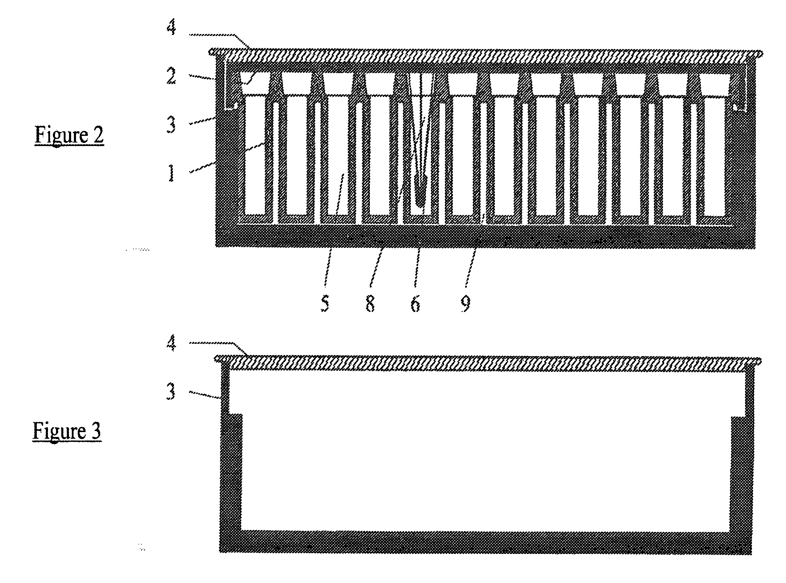

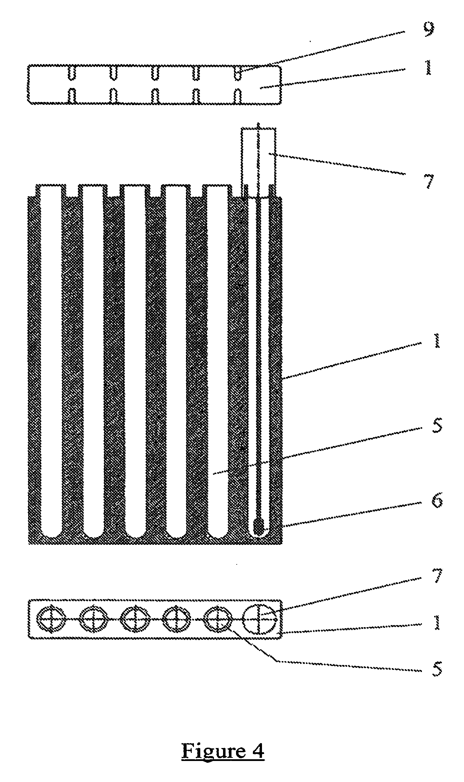

In the following embodiment of the present invention a preserving container in form of a storage box for 8 x 12 swabtips 8 is described in more detail with respect to the drawings. For this purpose it is referred to Figs. 1 to 3. A possible variation for storing shaft swabs 7 is shown in Fig. 4. According to Fig. 2 sample container 1 closed with lid 2 is positioned in storage container 3, which is covered by cover 4. In Fig. 3 a covered empty storage container 3 is shown.

Fig. 1 shows an opened sample container 1 having a fixedly mounted lid 2.

Sample container 1 and lid 2 materially consist of vapour permeable cardboard or paperboard, and storage container 3 as well as associated cover 4 are made of gas tight plastics. Alternatively, equivalents to cardboard, paperboard and plastics may be utilized.

Corresponding manufacturing methods of the components are not subject of this invention und are therefore left to the skilled person.

Lid 2 is mounted in a non-detachable way to sample container 1.

Sample container 1 is diverted into separated cavities 5. According to Fig. 2 cavities 5 are each provided for receiving a single swabtip 8. According to Fig. 4 they receive a shaft swab 7, respectively. In all cases cotton wool heads 6 do not come into contact with a wall- and bottom of sample container 1. In Fig. 4 the corresponding storage container 3 is omitted.

The design of sample container 1 allows the circulation of air between individual cavities 5. For this purpose clearances/gaps 9 in the container are provided.

### Reference list

- 1: Sample container,
- 2: Lid,
- 3: Storage container,
- 4: Cover,
- 5: Cavity,
- 6: Cotton wool head,
- 7: Shaft swab with plast-plug,
- 8: Swabtip,
- 9: Clearance/Gap.

## Claims

1. Preserving container, wherein a sample container (1) closable with a lid (2) is arranged in a storage container (3) provided with a sealing cover (4), the sample container (1) has separated cavities (5), in which sample carriers can be positioned without contacting any wall- and bottom of the sample container (1), the sample container (1) and lid (2) are made of diffusion permeable material and the storage container (3) as well as cover (4) are made of diffusion impermeable material.

2. Preserving container according to claim 1, **characterized in that** swabtips (8), alternatively also shaft swabs (7), can be arranged as sample carrier in the cavities (5) and that the sample container (1) is dimensionally adjusted, respectively, wherein also arrangements are provided, namely corresponding clearances (9) for a circulation of air between the individual cavities (5).

3. Preserving container according to claim 1, **characterized in that** the sample container (1) has at least one cavity (5), optimally 96, and also more than 96 cavities (5).

4. Preserving container according to claim 1, **characterized in that** all cavities (5) can be sealed using a central sealable lid (2).

5. Preserving container according to claim 1, **characterized in that** the storage container (3) comprises a plurality of sample containers (1).

6. Preserving container according to any of the preceding claims, wherein a humidity indicator is located within or on the container.

## Patentansprüche

1. Asservierungsbehälter, **dadurch gekennzeichnet, dass** ein mit einem Deckel (2) verschließbarer Probenbehälter (1) in einem mit einer dichtenden Abdeckung (4) versehenem Lagerbehälter (3) angeordnet ist, dass der Probenbehälter (1) voneinander getrennte Hohlräume (5) aufweist, in die Probenträger ohne Wand- und Bodenberührung mit dem Probenbehälter (1) angeordnet werden können, und dass der Probenbehälter (1) und der Deckel (2) aus diffusionsdurchlässigem Material und der Lagerbehälter (3) sowie die Abdeckung (4) aus diffusionsundurchlässigem Material hergestellt sind.

2. Asservierungsbehälter nach Anspruch 1, **dadurch gekennzeichnet, dass** als Probenträger Tupfer (8), alternativ auch Stielwattetupfer (7), in den Hohlräumen (5) angeordnet werden können und dass der Probenbehälter (1) dazu hinsichtlich seiner Abmessungen angepasst ist, wobei auch Vorkehrungen, nämlich entsprechende Zwischenräume (9), zur Luftzirkulation zwischen den einzelnen Hohlräumen (5) getroffen sind.

3. Asservierungsbehälter nach Anspruch 1, **dadurch gekennzeichnet, dass** der Probenbehälter (1) mindestens einen Hohlraum (5), optimal 96 oder auch mehr Hohlräume (5) aufweist.

4. Asservierungsbehälter nach Anspruch 1, **dadurch gekennzeichnet, dass** alle Hohlräume (5) mit einem zentralen versiegelbaren Deckel (2) verschließbar sind.

5. Asservierungsbehälter nach Anspruch 1, **dadurch gekennzeichnet, dass** der Lagerbehälter (3) mehrere Probenbehälter (1) umfasst.

6. Asservierungsbehälter nach einem der vorhergehenden Ansprüche, worin ein Feuchtigkeitsindikator in oder auf dem Behälter angeordnet ist.

## Revendications

1. Récipient de protection, dans lequel un récipient à échantillon (1), susceptible d'être fermé avec un couvercle (2), est agencé dans un récipient de stockage (3) muni d'un couvercle de fermeture étanche (4), le récipient à échantillon (1) comprenant des cavités (5) séparées, dans lesquelles des porte-échantillons peuvent être positionnés, sans entrer en contact avec aucune paroi et fond du récipient à échantillon (1), le récipient à échantillon (1) et le couvercle (2) sont composés d'un matériau perméable à la diffusion et le récipient de stockage (3), ainsi que le couvercle (4), sont composés de matériau imperméable à la diffusion.

2. Récipient de protection selon la revendication 1, **caractérisé en ce que** des coton-tiges (8), en variante également des écouvillons à tige (7), peuvent être agencés, à titre de support d'échantillon, dans les cavités (5), et **en ce que** le récipient à échantillon (1) est de dimensions ajustées, respectivement, dans lequel également des agencements sont prévus, précisément des jeux (9) correspondant, pour permettre une circulation d'air entre les cavités (5) individuelles.

3. Récipient de protection selon la revendication 1, **caractérisé en ce que** le récipient à échantillon (1) présente au moins une cavité (5), de manière optimale en présente 96, et également plus de 96 cavités (5).

4. Récipient de protection selon la revendication 1, **caractérisé en ce que** toutes les cavités (5) peuvent être fermées de manière étanche en utilisant un couvercle de fermeture étanche (2) central.

5. Récipient de protection selon la revendication 1, **caractérisé en ce que** le récipient de stockage (3) comprend une pluralité de récipients à échantillon (1).

6. Récipient de protection selon l'une quelconque des revendications précédentes, dans lequel un indicateur d'humidité est disposé à l'intérieur ou sur le récipient.
